Europäisches Patentamt

(19) European Patent Office

Office européen des brevets

(11) Publication number: **0 333 311 B1**

(12) # EUROPEAN PATENT SPECIFICATION

(45) Date of publication of patent specification:
04.12.91 Bulletin 91/49

(51) Int. Cl.⁵: **A61D 7/00, A61M 31/00**

(21) Application number: 89300832.6

(22) Date of filing: 27.01.89

(54) Intraruminal drug delivery device.

(30) Priority: 30.01.88 GB 8802099

(43) Date of publication of application:
20.09.89 Bulletin 89/38

(45) Publication of the grant of the patent:
04.12.91 Bulletin 91/49

(84) Designated Contracting States:
AT BE CH DE ES FR GB GR IT LI LU NL SE

(56) References cited:
EP-A- 0 025 699
EP-A- 0 164 927
GB-A- 2 155 335

(73) Proprietor: COOPERS ANIMAL HEALTH LIMITED
Berkhamsted Hill
Berkhamsted Hertfordshire HP4 2QE (GB)

(72) Inventor: Shepherd, Michael Trevor
Coopers Animal Health Limited Berkhamsted Hill
Berkhamsted Hertfordshire (GB)
Inventor: Watts, John
Coopers Animal Health Limited Berkhamsted Hill
Berkhamsted Hertfordshire (GB)

(74) Representative: Matthews, Derek Peter et al
Frank B. Dehn & Co. Imperial House 15-19 Kingsway
London WC2B 6UZ (GB)

## Description

The present invention relates to an intra ruminal device which has sufficient density to be retained in the rumen, a metal weight giving the required density to the device. Such a device is disclosed in the EP-A-0164927. By "retained in the rumen", is meant that the device is retained in the rumen of a normal healthy ruminant on a typical diet.

Conventional methods for preventing a ruminant from regurgitating an intraruminal device are either to make the device assume a shape in the rumen which prevents it passing back up the oesophagus (see for example European Patent Application 174865) or to make it sufficiently dense such that it sinks to the bottom of the rumen (see for example European Patent Applications 149510 and 164241). The density of the device can be made sufficiently high by incorporating a material of high mass normally a metal such as iron, which may either be in the form of a solid block or in the form of powder or shot which is contained within some restraining means, or distributed throughout the formulation. When the weight is provided in the form of powder or shot this is less dense and hence either leads to regurgitation or the need for a larger formulation than would be required for a formulation containing a solid mass of the same material. Where a metal is provided as a solid mass, this will normally have been cast or extruded into blocks and then machined to size, or cast directly into its desired shape or poured into a mould as a powder which is compressed into its unsintered form of the desired shape and then sintered in a furnace. Unfortunately, those formulations which incorporate the weight as a hard solid mass suffer from the disadvantage that this mass may not be compatible with the machinery utilised in meat processing. In particular the hard mass will often damage the meat works machinary for example, knives, guillotines or rollers used to cut the meat.

In powder metallurgy, metal powder is poured into a mould, often with suitable organic or inorganic excipients, and then compressed into shape. The metal "tablet" so formed is bound together sufficiently tightly to retain its shape but is friable and prone to disintegration on impact. This unsintered or "green" metal tablet is then sintered by heating to a high temperature in a furnace. The metal is then utilised in its sintered state and, in general, little use has been made of metal in its green or unsintered form.

It has now been discovered that if a formulation is retained in the rumen by means of a solid mass of unsintered or green metal, then the disadvantage of existing formulations of solid mass can be avoided. However, unsintered metal weight still retains the advantage of greater density over powder or shot formulations.

Accordingly, the solution to the problem to be solved is, that the metal weight comprises compacted unsintered metal.

The device is preferably an intra-ruminal bolus.

The metal weight may conveniently be in the form of a solid mass of unsintered metal, for example a cylindrical metal weight, or between two and fifty solid masses, typically between two and ten solid masses, which abut each other and form a single solid mass, or in the form of a tube of unsintered metal into which the active ingredient is filled or in the form of a piston provided in the bolus for expelling the active ingredient. In preferred embodiments of this invention, the metal weight is in the form of substantially cylindrical metal weight (or between two and ten masses which together form a substantially cylindrical metal weight); if desired the cylindrical weight may be domed or shaped at one or both ends, for example, to facilitate passage down the oesophagus of an animal.

The metal weight may be selected from any metal of sufficient density, or mixtures of these, such as iron, zinc, nickel or tungsten or may be an appropriate alloy. Conveniently, the metal is iron. The metal weight may contain binders for example magnesium stearate, conveniently used in the manufacturing of unsintered metal and sintered products. The metal weight conveniently has a density of between 5.0 and 13.0 $g.cm^{-3}$ and normally between 5.5 and 9.0 $g.cm^{-3}$. The device conveniently has an initial density of greater than 2.5 $g.cm^{-3}$.

When the metal is in its unsintered form, the bonds between the metal powder formed by the compression process are sufficient for it to retain its integrity when incorporated in a bolus but the metal is friable if handled roughly and is liable to fracture if dropped on a hard surface. Accordingly, if the weight is in an exposed position in the device, for example if it is provided as an end-weight or as a tube, then it is preferably enclosed or partially enclosed with a material, such as a plastics material, that prevents damage to the weight from rough handling and contains any broken pieces that may arise from rough handling. If the weight is provided within the device, for example as described in European Patent Application 149510 or as a piston within the device, external coating is optional. The friability of the metal weight is dependent on the compression forces and the binders used in the preparation of the metal "tablet". Thus, the friability of the metal weight may be adjusted to suit the purpose for which the weight is to be used and the process used for its preparation. Any coating of the metal weight will be sufficiently thin, soft or malleable to allow processing of the device in meatworks.

When the unsintered metal weight is on the exterior of the device and there is another metal present within the device with which the metal weight may form a galvanic couple, then the weight is conveniently coated with an electrically conducting material such as iron (which is conveniently applied by flame spraying, electrostatic deposition, dipping or painting), which both helps to protect the unsintered metal from damage and provides a surface electrode. Alternatively the unsintered metal weight is encased in a conductive plastics material which is coated with flame sprayed iron. Conveniently, the second metal is a magnesium alloy and corrodes in a manner that enables controlled release of a medicament from the device (see for example European Patent Application 0205342). The degradable second metal may be replaced by an alternative material degradable or dissolvable in the ruminal fluids, for example soluble glass, if desired. In such a case, there will not normally be a galvanic couple and coating of the metal weight to give an electrically conductive surface is not necessary.

The devices of the present invention are intended for oral administration to ruminants and their dimensions will be such as to allow them to pass freely down the animal's oesophagus into the animal's rumeno-reticular sac whilst being of sufficient size to enable incorporation of adequate quantities of active agent. Thus, convenient dimensions of devices for administration to cattle are between 10 and 45 mm in diameter, typically 25 mm in diameter, and 20 and 150 mm in length, typically 100 mm in length, whilst those for sheep are between 5 and 30 mm diameter, typically 20 mm, and 10 and 100 mm, typically 45 mm, length.

The active agents for incorporation in the devices of the present invention may be selected from therapeutic agents, growth promotants, vaccine formulations, nutrients, agents affecting fertility and other substances necessary for the physical well being of the ruminant.

Examples of therapeutic agents for _in vivo_ use include anti-infectives, e.g., an antiviral agent such as acyclovir, idoxviridine or vidarabine ; anti-bacterial agents such as penicillins, tetracyclines, erythromycin, neomycin, polymyxin B, gentamycin, nystatin, benzylpyrimidines such as trimethoprim or baquiloprim optionally in combination with a sulphonamide such as sulphadiazine, sulphadimidine, sulphadoxine or sulphadimethoxine, or bacitracin ; and anti-protozoals such as anti-coccidials.

Active agents for use in the bolus of the present invention also include anti-parasitic agents such as anthelmintics, for example oxfendazole (i.e. 2-methoxycarbonylamino-5-phenylsulphinylbenzimid azole), oxibendazole, parbendazole, niridazole, mebendazole, fenbendazole, cambendazole, albendazole, metronidazole, thiabendazole, levamisole, tetramisole, closantel, bromoxanide, rafoxanide, clioxanide, oxyclozanide, salantel, morantel, resorantel, pyrantel, praziquantel, febantel, oxantel, carbantel, piperazine, nicolosamide, brotianide, thiophanate, bephenium, pyrvinium, diethylcarbamazine, suramin, dichlorophen, paromomycin, stibophen, antimony sodium dimercaptosuccinate, hycanthone, metrifonate, antimony barium tartrate, antimony potassium tartrate, chloroquine, emethine, bithionol, hexylresorcinol, tetrachloroethylene, mirasan, miracil, lucanthone, furapromidium, oxamniquine, tubercidin, amphotalide, nicarbazin, Hetol (Trade Name), Hetolin (Trade Name), nitroxynil, disophenol, Bitin-S (Trade Name), bromofenophos, menichlopolan, thiosalicylanilide, diamphenethide, bunamidine, bitoscanate, nitroscanate, amoscanate, diuredosan, arsenamide, thiazothienol, thiazothielite, haloxon, dithiazanide iodide, bidimazium iodide, methyridine dymanthine trichlabendazole, chlorsulan and avermectins such as ivermectin.

Nutrients or other substances conducive to physical health, for use in the bolus according to the present invention, include vitamins and agents which have a vitamin-like physiological role. Vitamin mixes, particularly containing one or more B vitamins, may be included for the treatment of fodder poisoning or acute bacterial toxaemia in animals. For the assistance of rehydration in fluid-electrolyte loss associated with scours in calves, lambs and pigs, the biologically active material in the bolus of the invention may comprise a composition of suitable electrolytes including sodium chloride, and/or one or more appropriate sugars, for example glucose. Particularly when intended for administration to grazing animals, such nutrients may include trace elements, for example selected from magnesium, zinc, copper, cobalt and selenium.

When the device contains a degradable metal this is conveniently selected from magnesium, aluminium, zinc, titanium and their alloys, an alloy of magnesium and one or more other metals is preferred, for example selected from the following (all percentages being by weight) : silicon up to 1.5%, nickel up to 1%, zinc up to 15% (for example about 12%), manganese up to 2%, copper up to 4% (for example about 2% or about 3%), aluminium up to 15% for example about 12%), and zirconium up to 0.8%. It will be appreciated by those skilled in the art that not all of the above metals are mutually compatible.

Other active agents for use in the device of the present invention include growth promotants such as tetronasin, lasalocid, monensin, virginiamycin, zinc bacitracin, flavomycin, avoparcin, nitrovin, thiopeptin, halquinol, carbadox, tylosin and arsanilic acid (including carbarsone) or combinations of these. Some of these agents (e.g. zinc bacitracin) are also antibacterial agents.

For enhancing the fertility of animals or inducing ovulation, one or more appropriate hormone or hormone mimetics such as prostalene, optionally in combination with vitamin E, may be included.

In particular, therapeutic agents may be provided in discrete units such as capsules, cachets or tablets, each containing a predetermined amount of agent, as a powder or granules. Such formulations may be prepared by any of the methods of pharmacy and may include the step of bringing into association the agent with a binder and/or carrier which constitutes one or more accessory ingredients. In general they are prepared by uniformly and intimately admixing the agent with liquid or finely divided solid carriers or both, and then, if necessary, shaping the product into the desired presentation. For example, a tablet may be prepared by compression or moulding a powder or granules of the agent, optionally with one or more accessory ingredients. Compressed tablets may be prepared by compressing, in a suitable machine, the agent in a freeflowing form such as a powder or granules optionally mixed with a binder, lubricant, inert diluent, surface active or dispersing agent(s). Moulded tablets may be made by moulding, in a suitable machine, the powdered agent moistened with an inert liquid diluent. Alternatively, such formulation may be prepared by admixture of the agent with one or more of the conventional solid carriers, for example cocoa butter, and shaping of the resulting mixture.

When the therapeutic agent is formulated as a tablet, the tablet may have, for example, especially for administration to cattle, a weight in the range of 0.5 to 5 g, for example 0.75 to 2 g, typically about 1 g. When the tablet is provided with a central hole through which extends a central rod, typically this may have a diameter in the range of from 4 to 15 mm. The weight of active ingredient in the tablet may for example be in the range of from 50 to 95% w/w, typically about 75% w/w. Suitable binders, carriers and other diluents include disintegrants such as starch, e.g. 5 to 15%, typically 10% w/w, binders such as polyvinylpyrrolidene (PVP), e.g. 0.5 to 5% w/w, typically 2.5% w/w and lubricants such as magnesium stearate, e.g. 0.2 to 2.5% w/w, typically 1.0% w/w. In any tablet formulation, if desired, the remaining bulk of the tablet may be made up with a material such as lactose. It will be appreciated that such compositions of therapeutic agent may also be formulated as a cylinder when intended for use in a bolus having a single or multiple segments of therapeutic agent. Such a cylinder may have a weight of e.g. 5 to 100 g, typically about 80 g, a diameter from 4 to 35 mm, typically about 22 mm and an overall length of e.g. 20 to 150 mm, typically about 100 mm. The weight may also be incorporated as a discrete mass within the drug matrix. Such a matrix conveniently comprises, monostearin, carnuba wax and barite and may have a weight of e.g. 10 to 300 g, typically about 100 g for cattle, a diameter of 10 to 45 mm, typically about 22 mm for cattle, and an overall length of e.g. 10 to 150 mm, typically about 100 mm for cattle.

The boluses of the present invention will be assembled by standard production techniques, e.g. forming the casing by standard techniques, such as thermoforming, and loading the weight and therapeutic agents and sealing the bolus by conventional means, e.g. transfer moulding, insert moulding or injection moulding.

The present invention will now be described in more detail by way of the following exemplary embodiments and with reference to the accompanying drawings in which :

Figure 1 shows in longitudinal section a bolus according to the present invention for the pulsed release of an active agent.

Figures 2, 3 and 4 show in longitudinal section further variants of a bolus according to the present invention for the substantially constant release of an active agent.

Figure 5 shows in longitudinal section a further variant of a bolus according to the present invention for delivering increasing amounts of an active agent.

Figures 6 and 8 show in longitudinal section further variants of a bolus according to the present invention for the pulsed release of an active agent.

Figures 7 and 9 show in cross-section through A-A and B-B the boluses shown in Figures 6 and 8 respectively.

Figures 10 and 11, 12 show in longitudinal section further variants of boluses according to the invention for substantially constant release of an active agent.

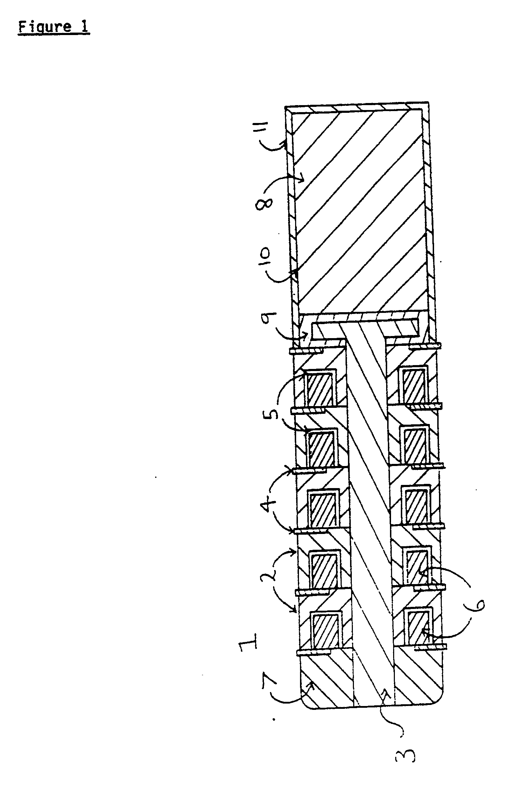

Figure 1 shows a bolus 1 comprising a plurality of UPVC segments 2 mounted by interference fit on a central rod 3 of biodegradable material, for example magnesium alloy or soluble glass, passing through apertures in the centres of the segments. A seal is created between adjacent segments by silicone rubber washers 4. Respective annular cavities 5 are provided in each of the segments to have annular tablets 6 containing the active agent. At the front of the device a blank UPVC segment 7 is located by interference fit over the rod which extends through an aperture in the centre of the blank segment. At the rear of the device, a cylindrical mass of compacted unsintered metal 8 abuts a polypropylene disc 9 which encases one end of the rod, the external face of the disc and the metal mass being encased in a conductive polypropylene sheath 10 which is bonded to the polypropylene disc, for example by welding or adhesives. The polypropylene sheath is provided with a surface coating of iron 11 when the rod is constructed of magnesium alloy.

In use, when the device is administered to an animal and the rod is constructed of magnesium alloy, a galvanic couple is established between the magnesium rod and the iron surface coating which promotes corrosion of the rod until the blank segment and adjacent washer fall away and are eventually released. The first tablet of the bolus is then exposed and released. Corrosion of the rod continues with successive shedding of segments and washers and subsequent release of tablets until all the tablets are released. As the galvanic couple is constant whilst the rod is degrading the rod degrades at a uniform rate and the tablets are released at constant time intervals. The shed segments and washers are not sufficiently dense to be retained in the rumen and are regurgitated or excreted. The unsintered metal mass remains in its casing in the rumen but because of the nature of unsintered metal it does not interfere with the machinery of the meat processing works when the animal is slaughtered, i.e. knives or guillotines will break the unsintered metal whilst rollers will crush it. When the rod is constructed of soluble glass this dissolves at a constant rate releasing the tablets as before.

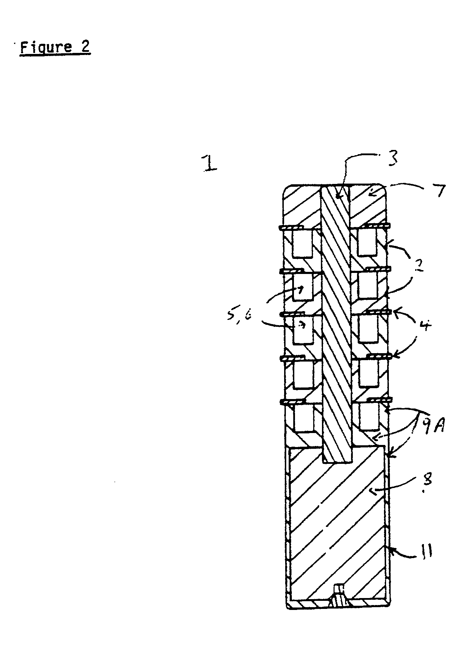

In an alternative embodiment of the bolus of Figure 1 the tablet segment adjacent to the metal weight, the polypropylene disc and the polypropylene sheath are a single unit (9A) formed around the weight and the base of the core by insert moulding. This embodiment is illustrated in Figure 2.

In a further alternative to the boluses shown in figures 1 and 2 one or more of the tablets of active agent is replaced by a tablet (or tablets) of green metal.

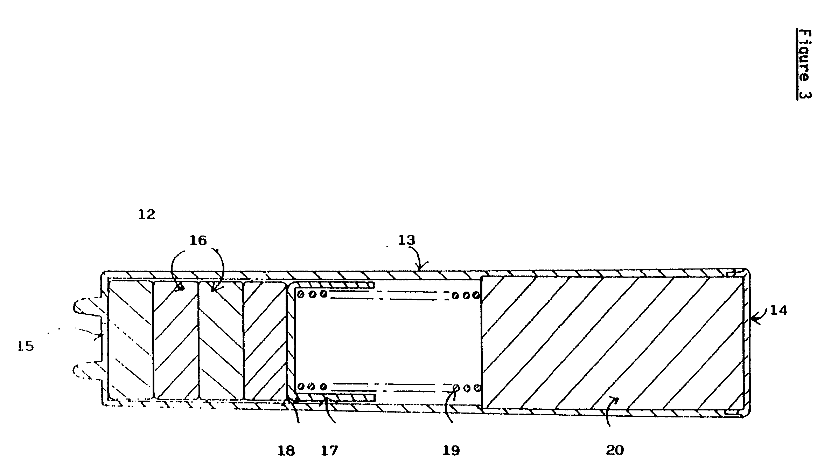

Figure 3 shows a bolus 12 having a cylindrical polypropylene casing 13 with a polypropylene closure 14 snap-fitted and sealed at the rear end. The casing partially encloses the front end to provide a circular hole 15 through which active agent may be expelled from the bolus. The active agent is contained in tablets 16 which degrade on exposure to the rumen. For constant release of active agent, the active agent will be uniformally distributed in each tablet and amongst the tablets. The leading face of the tablets is kept pressed against the circular hole by means of a polypropylene piston 17 which is in contact with the rear face of the tablet 18 and has a compressed spring 19 acting upon it. A cylindrical mass of unsintered metal 20 is provided behind the spring and fills the remaining portion of the bolus. The interior wall of the casing is of slightly greater circumference around the metal mass than elsewhere forming a lip for the metal mass to abut against. The metal forms a slide fit with the wall of the casing. Thus, even when the compression spring is at its fuller extension within the bolus the metal mass will not move within the bolus.

In use, the bolus is administered to an animal and resides in the animal's rumeno-reticular sac. The exposed leading face of the tablets embibes liquid, extrudes, erodes and releases the active agent. As the tablets erode, the bias provided by the sprung piston keeps the leading edge in contact with the hole in the casing until, ultimately, all the tablets have eroded. Then, as in the device illustrated in Figure 1, the bolus is retained in the rumen but does not damage the meat processing works machinery when the animal is slaughtered.

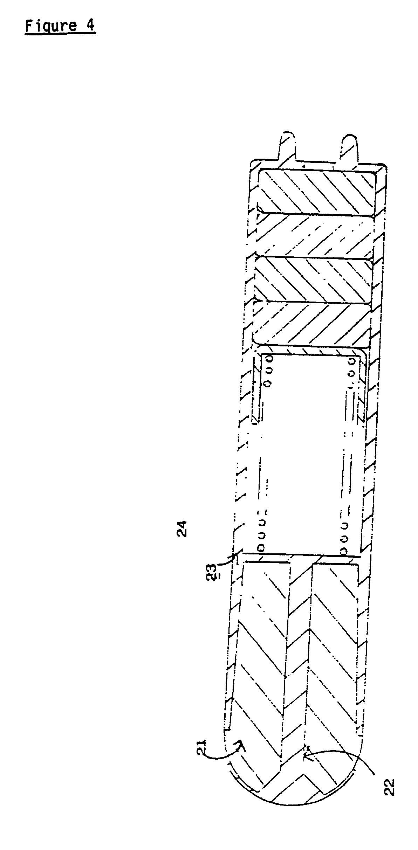

Figure 4 shows a variant of the bolus shown in Figure 3 in which the end of the mass of unsintered metal 21 at the rear of the device is dome-shaped. The metal mass has a annular hole 22 which widens out at the domed end and the metal mass is retained in place by means of a moulded polypropylene shaft integral with the casing 23 of the bolus 24. In this case, the metal mass does not have to be totally encased in the casing although some protection of the metal mass by the casing is preferred and is shown in the figure.

In use, the bolus releases active agent as described for the device illustrated in Figure 2.

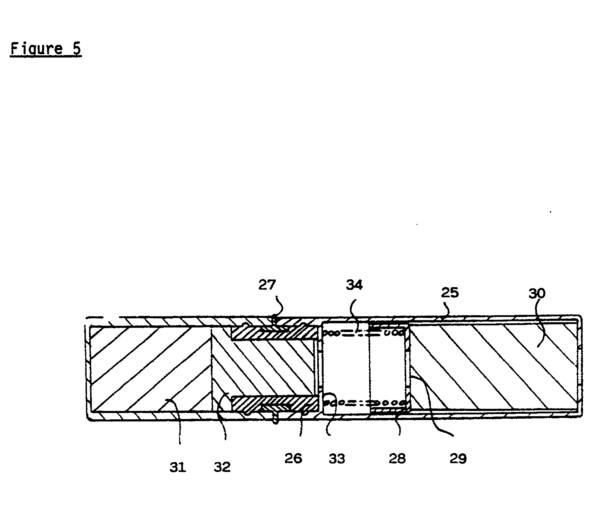

Figure 5 shows a further variant of the bolus shown in Figure 2 in which the cylindrical polypropylene casing 25 is in two sections joined together via an internal "O"-ring 26 constructed of plastics material, such as polyox, PLA, PGA, Biopol, etc. which is biodegradable in the ruminal fluids. A rubber seal 27 prevents premature ingress of the ruminal fluids. A piston 28 is provided with an aperture 29 to permit ingress of the ruminal fluids into the device once active agent 30 has been expelled. The green metal weight is in the form of two segments 31 and 32 which abut an internal retaining wall 33 against which a spring 34 is biased. The internal retaining wall is provided with one or more apertures to permit the ruminal fluids to come into contact with the "O"-ring once these pass through the piston.

In use, the active agent erodes as in the device of figure 3 until it is expelled and the piston is biased against the front end of the device. Ruminal fluids seap into the device through the aperture in the piston and pass through the internal retaining wall. The "O"-ring degrades in the ruminal fluids, the polypropylene casing splits into its two sections and the two segments of the green metal weight fall out of the casing. The two sections of the casing which contain the piston and spring together with the rubber seal are regurgitated by the animal because of their low density whilst the segments of the green metal weight either remain in the rumen or are eroded in the rumen, the combined abrasive action of the segments assisting in this process. The boluses of figures 1, 2, 4, 6, 7 and 9 may similarly have a two-part casing separated by a rubber seal and O-ring so that they disintegrate in the rumen as described for the bolus of figure 5.

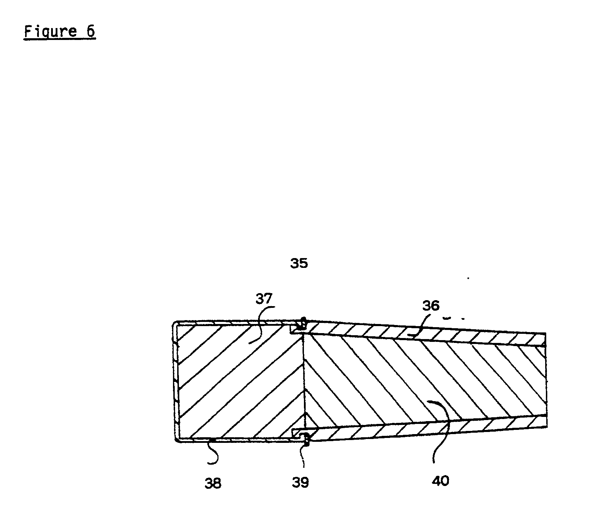

Figure 6 shows a bolus 35 comprising a frusto-conical tube 36 formed of magnesium alloy or soluble glass the rear end of which is fixedly located by interference fit into a green metal weight 37. The green metal weight is encased in a plastic casing 38 which will be of conductive material when the tube is constructed of magnesium, a rubber washer 39 sealing the join between the tube and casing. A core 40 contains an active agent dispersed in a suitable excipient with an appropriate binder. The core is tapered so as to fit exactly into the cavity defined by the green metal weight and the interior surface of the tube.

The exterior surface of the tube, other than its leading edge, is coated with a liquid impermeable coating, for example aluminium oxide or epoxy resin, whilst the conductive plastic casing carries an iron spray coating when the tube is constructed of magnesium.

The bolus is constructed by forming the tube, applying the surface coating and then inserting the core. The green metal weight is fitted to the tube with the rubber sealing washer in place and the conductive plastic casing moulded around the metal. The plastic coating is then sprayed with zinc bondcoat and then iron.

In use, the bolus is administered to a calf by an oesophageal balling gun, after which it resides in the animal's rumeno-reticular sac. The exposed core surface is subject to attack by the ruminal fluids and begins to release the active agent. The core thereby starts to erode or dissolve so that the exposed surface retreats towards the end weight. At the same time, the end face of the tube also exposed to the ruminal fluids and dissolves. When the tube is constructed of magnesium direct electrical contact between the tube and the iron coating on the green metal weight casing sets-up a galvanic couple. Since magnesium is less noble than iron, the magnesium alloy also begins to corrode in the rumenal fluid. As the tube progressively corrodes or dissolves from the end face thereof, a portion of surface coating remains unsupported, but is sufficiently thin and brittle so as to fragment fairly quickly and thus break off, so that the coating does not extend too much beyond the tube end face. Moreover, the compositions of the core and tube are chosen such that the exposed surface of the core and the end face of the tube retreat towards the endweight at generally the same rate, the two surfaces thus remaining more or less co-planar.

The casing along the length of the core, that is to say the tube with its coatings which are outside the endweight, progressively corrodes only from the open front end. As degradation of the bolus progresses, the diameter of exposed core surface increases so that the amount of the active agent released per unit time also increases.

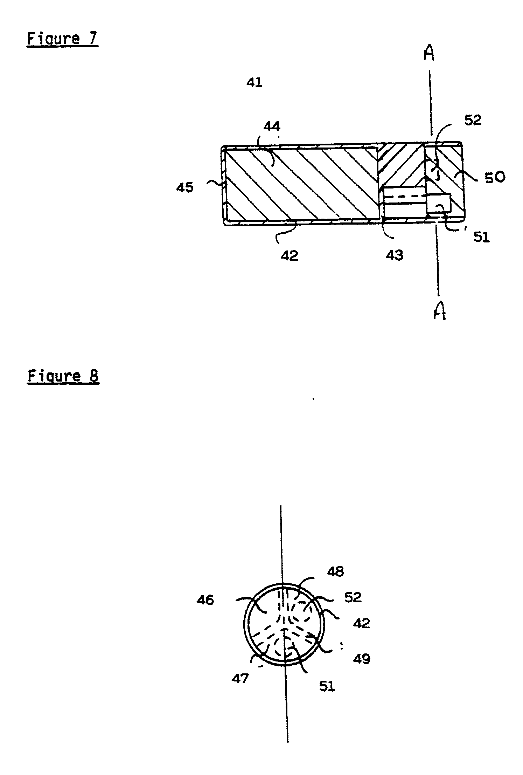

Figures 7 and 8 show a bolus 41 for pulsed released of an active agent comprising a plastic cylindrical casing 42 (for example of conductive polypropylene) separated into two sections by a dividing wall 43. A green metal weight 44 is contained in one section and retained by a plastics endplate 45 ultrasonically welded to the casing. One or more active agents dispersed in a suitable excipient are contained in discrete compartments 46, 47, 48 in the other section, the compartments being separated by a plastic wall 49 sealed into the section, for example by epoxy resin. A cylindrical cap 50 constructed of material degradable by the ruminal fluids seals the end of the bolus. The cap is provided with recesses 51, 52 in its interior surface, each recess being of different depth and being positioned so that it is aligned over a compartment (the cap does not need to be recessed in relation to one compartment).

When the cap is constructed of magnesium alloy, the plastic coating will be constructed from a conductive polymer and spray coated with zinc bondcoat and iron as described for the previous boluses, to create a galvanic couple in the rumen. If the cap is constructed of a non-metallic degradable material, for example soluble glass, then the need for the casing to be constructed from a conductive polymer which is spray coated is eliminated.

In use, the bolus is administered to the ruminant such that it resides in the animal's rumeno-reticular sac. The cap degrades progressively in the ruminal fluids so that the compartments are exposed sequentially to the rumen fluids releasing active agent into the rumen. The time between exposure of each compartment and consequent "pulse release" of active agent into the rumen is dependent on the thickness of the cap covering the compartment, or, conversely, the depth of the recess in the cap aligned over that compartment.

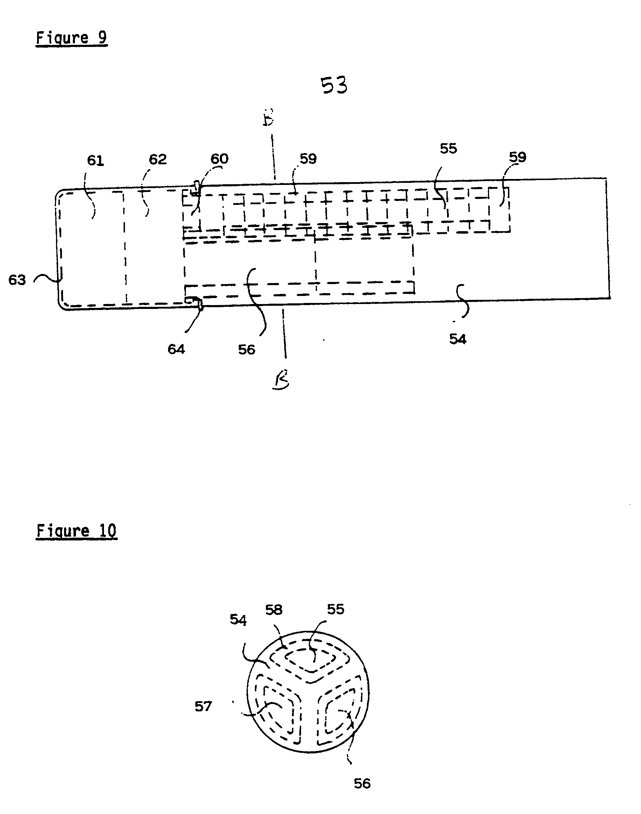

Figures 9 and 10 shows a bolus 53 a variation of the bolus shown in Figures 6 and 7 in which the casing, compartment wall and end cap are formed of a one piece cylindrical magnesium block 54 in which cavities 55, 56 and 57 of varying depths have been drilled. The cavities contain the active agent dispersed in a suitable excipient and are lined with plastics materials 58, which may be an epoxy resin coating or a series of plastic or rubber components which abut each other, and provided with plastics plugs 59, 60 at each end. The curved surface of the magnesium block is provided with an aluminium oxide protective coating. A green metal endweight (or weights 61, 62) is retained in a conductive plastic sheath 63 provided with an iron sprayed coating. This is joined to the magnesium block by intereference fit. A rubber seal 64 is provided between the block and sheath. As in the boluses described in figures 1, 2, 6 and 7, the magnesium block may be replaced by a soluble glass block.

In use, the magnesium degrades in the rumen from its exposed end, active agent being released from each cavity sequentially as the end face of this is exposed. The plastics materials lining retreats with the exposed face of the magnesium block.

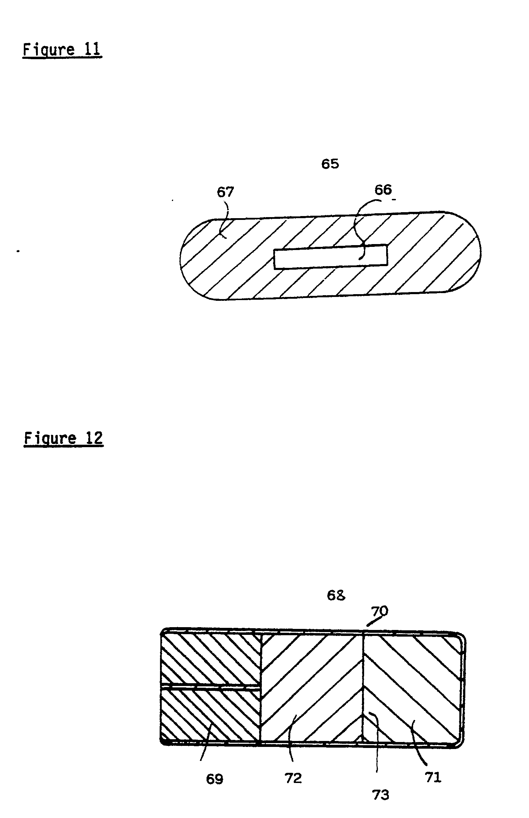

Figure 11 shows a bolus 65 having a green-metal core 66 surrounded by active agent dispersed in an excipient 67 which has been transfer moulded around the core. The excipient typically comprises a mixture of monostearin, carnuba wax and barium sulphate. The bolus is administered to the ruminant and is retained in the rumen by its high density (initial density of device shown 2.7 g. cm$^{-3}$). The excipient erodes in the rumen releasing active ingredient until only the metal core is left (density 6.5 g. cm$^{-3}$).

Figure 12 shows a bolus 68 having an annular unsintered metal weight 69 retained at the open end of a plastic casing 70 semi-permeable to the ruminal fluids, for example a cellulose acylate. The metal weight is provided with a plastic protective surround that maintains the integrity of the metal weight. A water-swellable material 71, for example hydrogel, is provided at the other end of the casing and a thermosensitive composition 72 containing the active agent is sandwiched between the weight and the water swellable material. A dividing wall 73 constructed for example from a waxy material such as a food grade petroleum based wax, separates the thermosensitive composition from the water swellable material.

The thermosensitive composition is solid at room temperature but becomes fluid at the temperature of the rumen. In use, ruminal fluids permeate the plastic casing of the bolus and cause the water swellable material to expand. The water swellable material thus exerts pressure through the dividing wall on the fluid thermosensitive composition expelling this from the device. The kinetics of the explusion of the thermosensitive composition are dependent inter alia on the viscosity of the composition, the oriface size and the nature of the water swellable material and the permeability of the casing (see for example US Patent 4717718).

## Claims

1. An intra-ruminal device which has sufficient density to be retained in the rumen, a metal weight giving the required density to the device ; characterised in that the metal weight comprises compacted unsintered metal.

2. A device according to claim 1 in which the metal weight is a substantially cylindrical metal weight formed of a solid mass of unsintered metal or between two and ten such masses.

3. A device according to claim 1 in which the metal weight is in the form of a tube.

4. A device according to any one of claims 1 to 3 in which the metal weight is on the exterior of the device and there is another metal present within the device with which the metal weight may form a galvanic couple, the metal weight being coated with an electrically conducting material or encased in a conductive plastics material coated with an electrically conducting material.

5. A device according to claim 4 in which the second metal is a magnesium alloy.

6. A device according to claim 1 in which the metal weight is an annular metal weight.

7. A device according to claim 1 in which the unsintered metal weight is incorporated as a discrete mass within a drug matrix.

8. A device according to any one of claims 1 to 7 adapted for the pulsed release of an active agent.

9. A device according to any one of claims 1 to 7 adapted for the constant release of an active agent.

10. An intra-ruminal device according to any one of claims 1 to 7 adapted for the administration of one or more active agents selected from the group comprising anthelmintics and growth promotants.

11. An intra-ruminal device according to claim 10 in which the active agent is oxfendazole and/or tetronasin.

## Patentansprüche

1. Vorrichtung für Wiederkäuer zum Einbringen in den Pansen, die eine ausreichende Dichte hat, um im Pansen zurückgehalten zu werden, wobei ein Metallgewicht der Vorrichtung die geforderte Dichte gibt, dadurch gekennzeichnet, daß das Metallgewicht gepreßtes bzw. kompaktes ungesintertes Metall umfaßt.

2. Vorrichtung nach Anspruch 1, bei der das Metallgewicht im wesentlichen ein zylindrisches Metallgewicht ist, das aus einer festen Masse von ungesintertem Metall oder aus zwei bis zehn solcher Massen gebildet ist.

3. Vorrichtung nach Anspruch 1, bei der das Metallgewicht die Form eines Rohres aufweist.

4. Vorrichtung nach einem der Ansprüche 1 bis 3, bei der das Metallgewicht außerhalb der Vorrichtung ist und wo ein anderes Metall innerhalb der Vorrichtung vorhanden ist, mit dem das Metallgewicht ein galvanisches Paar bilden kann, wobei das Metallgewicht mit einem elektrisch leitenden Material beschichtet ist oder in ein leitendes Kunststoffmaterial eingebaut ist, das mit einem elektrisch leitenden Material beschichtet ist.

5. Vorrichtung nach Anspruch 4, bei der das zweite Metall eine Magnesiumlegierung ist.

6. Vorrichtung nach Anspruch 1, bei der das Metallgewicht ein ringförmiges Metallgewicht ist.

7. Vorrichtung nach Anspruch 1, bei der das ungesinterte Metallgewicht als eine diskrete Masse innerhalb einer Medikamentmatrix eingebaut ist.

8. Vorrichtung nach einem der Ansprüche 1 bis 7, die für eine gepulste Abgabe eines aktiven Wirkstoffes geeignet ist.

9. Vorrichtung nach einem der Ansprüche 1 bis 7, die für eine gleichmäßige Abgabe eines aktiven Wirkstoffes geeignet ist.

10. Vorrichtung für Wiederkäuer nach einem der Ansprüche 1 bis 7, die zum Verabreichen von einem oder mehreren aktiven Wirkstoffen ausgewählt aus der Gruppe umfassend Wurmmittel und Wachstumsförderer geeignet ist.

11. Vorrichtung für Wiederkäuer nach Anspruch 10, bei der der aktive Wirkstoff Oxfendazol und/oder Tetronasin ist.

## Revendications

1. Dispositif intra-ruménal, qui possède suffisamment de densité pour être retenu dans le rumen, une charge de métal conférant la densité voulue au dispositif, caractérisé en ce que la charge de métal est constituée d'un métal tassé ou compacté, non fritté.

2. Dispositif suivant la revendication 1, caractérisé en ce que la charge de métal est constituée par une charge de métal, sensiblement cylindrique, formé d'une masse solide d'un métal non fritté ou par deux à dix de masses de ce genre.

3. Dispositif selon la revendication 1, caracterisé en ce que la charge de métal se présente sous la forme d'un tube.

4. Dispositif suivant l'une quelconque des revendications 1 à 3, caractérisé en ce que la charge de métal se trouve à l'extérieur du dispositif et en ce qu'un autre métal est présent dans le dispositif, à l'aide duquel la charge de métal peut former un couple galvanique, la charge de métal étant enrobée d'une matière conductrice de l'électricité, ou noyée dans une matière plastique conductrice, enrobée d'une matière conductrice de l'électricité.

5. Dispositif suivant la revendication 4, caractérisé en ce que le second métal est constitué par un alliage de magnésium.

6. Dispositif suivant la revendication 1, caractérisé en ce que la charge de métal est une charge de métal annulaire.

7. Dispositif suivant la revendication 1, caractérisé en ce que la charge de métal non frittée est incorporée sous forme de masse distincte dans une matrice de médicament.

8. Dispositif suivant l'une quelconque des revendications 1 à 7, adapté à la libération pulsée d'un agent actif.

9. Dispositif suivant l'une quelconque des revendications 1 à 7, adapté à une libération constante d'un agent actif.

10. Dispositif intra-ruménal suivant l'une quelconque des revendications 1 à 7, adapté à l'administration d'un ou plusieurs agents actifs choisis dans le groupe formé par les composés anthelmintiques et les facteurs de croissance ou agents qui favorisent la croissance.

11. Dispositif intra-ruménal suivant la revendication 10, caractérisé en ce que l'agent actif est l'oxfendazole et/ou le tétronasin.

Figure 1

Figure 2

Figure 3

Figure 4

Figure 5

Figure 6

Figure 7

Figure 8

Figure 9

53

Figure 10

Figure 11

65

67

66

Figure 12

68

70

69    72    71

73